# EUROPEAN PATENT SPECIFICATION

(11) **EP 0 735 849 B1**
(45) Date of publication and mention of the grant of the patent: **20.03.2002**
(21) Application number: 95905099.8
(22) Date of filing: 21.12.1994
(51) Int. Cl.: A61F 13/15, A61L 15/28

(54) **THIN SANITARY PRODUCTS WITH A PRE-FABRICATED ABSORBENT BODY**
DÜNNER HYGIENEARTIKEL MIT EINEM VORGEFERTIGTEN ABSORBIERENDEN KÖRPER
ARTICLES HYGIENIQUES MINCES COMPORTANT UN CORPS ABSORBANT PREFABRIQUE

(30) Priority: 22.12.1993 DE 4343947
(43) Date of publication of application: 09.10.1996
(73) Proprietor: THE PROCTER & GAMBLE COMPANY, Cincinnati, Ohio 45202 (US)
(72) Inventor: ZIMMERMANN, Volker, D-91301 Forchheim (DE); SCHÖNEBAUM, Andreas, D-90429 Nürnberg (DE); FRITSCHE, Ulrich, D-90425 Nürnberg (DE)
(74) Representative: Kremer, Véronique
(86) International application number: EP9404263
(87) International publication number: WO9517147

(56) References cited:
- WO-A-91/15177
- DE-A- 3 045 225
- DE-A- 3 141 098
- US-A- 3 070 095
- US-A- 3 903 889
- US-A- 4 822 453

## Description

The invention relates to absorptive ultra thin sanitary products of a textile or nonwoven material and of a swellable, natural substance included therein.

Absorbent sanitary products (which, according to the invention, also mean medical products), for instance disposable products such as diapers, sanitary napkins, incontinence articles and surgical pads, contain as water-absorbing substances so-called super absorbers, i.e. absorbents having an absorptivity for water and body fluids.

As a rule, these super absorbers are based on synthetic polymers, in particular on acrylic- or methacrylic-acid-based, cross-linked synthetic polymers and copolymers. These known synthetic absorbents are virtually water insoluble, absorbing, when equilibrated, a multiple of their weight in water or other aequous solutions. Starch-based products are known as well, such as for instance starch acrylonitrile graft polymers, gelatinized starch derivates or cellulose-based derivates, such as for instance derivates of alkyl- or hydroxyalkylcellulose, carboxymethylcellulose and polysaccharide-based derivates.

Disadvantages of synthetic super absorbers
- absorptivity decreasing over prolonged periods of time
- in a swollen condition, susceptible to shearing forces
- susceptibility to highly osmolar solutions, in particular of bivalent ions.

Further, polyose-(hemicellulose-)based super absorbers are known, such as for instance absorbers based on galacto(gluco)mannans, and in particular guar gum. They have the advantage of being biodegradable as natural substances. They are genuine swellable substances without ion exchange function, however, such swellable substances with ion exchange function also exist.

Galacto(gluco)mannans have an extraordinarily high swellability; their swelling time being in the range of approximately 15 seconds, which however has the disadvantage that barrier layers may result from the rapid swelling and prevent the galactomannan particles not yet swollen from coming in touch with liquid, these particles no longer functioning as swellable material. A further disadvantage resides in that these substances swell only very little under pressure.

An absorbent and swelling agent for water, aequous solutions and body fluids is known from EP-A-0481225, which consists of a physical mixture of at least two components and in which a component A is at least one water swellable, synthetic polymer or copolymer cross-linked with a multifunctional compound, and the component B is at least one polysaccharide from the group of galactomannans or polygalactomannans as well as galactomannan derivates or their mixings with other natural or synthetic polymerides as exist as pourable powders or as fiber substances at normal temperatures and are only partially water soluble or insoluble. EP-A-0481225 solved the problem of comparatively low swellability under pressure by the addition of synthetic SAP.

US-A-3903889 teaches to employ a borate-anion-modified guar gum as a super absorber; borate anions are incorporated in the form of a substantially water insoluble, borate-release agent into the absorptive product, in which the released borate ion is then slowly yielded to the absorbing system, after the aequous liquid to be absorbed has entered into the product. The guar gum thus modified is alleged to absorb up to at least 20 times its weight of water, forming a relatively dry, non-sticky, and inert gel.

DE-C-3045225 specifies a method of producing an absorptive material, in which a cis-1,2-diol-polysaccharide and water are mixed for the material to be substantially totally hydratized, an aequous solution of a borate-ion-release compound then being added to this material, and the cross-linked, clodded, gelled material then being dehydrated.

Neither US-A-3903889 nor EP-A-0481225 solve the problem of low swellability under pressure. Either patent achieves the cross-linking of the polymer only in the weakly alkaline medium.

US-A-3 070 095 discloses a multi-layer napkin, such as a disposable diaper or the like. An intermediate layer of this napkin is the principal water-absorbent portion of the product. The water-absorption by the intermediate layer is done essentially by discrete particles of a dry water-absorbent substance. This can be of organic source and of synthetic or natural origin. The substance can be supplied to a prefabricated cellulose-type sheet of material, by passing the sheet under a sifting feeder for the finely divided water-absorbent substance. The substance is distributed over the top surface of the sheet as it passes horizontally under the sifter.

DE-A-3 141 098 refers to an absorbent material for aqueous body fluids. The absorbing substance comprises a carrier, on which a synthetic or natural swellable polymer or co-polymer is stably fixed by applying an at least partly swollen polymer or co-polymer to the carrier and subsequent drying of the latter.

WO-A-91 15 177 discloses absorbent members containing interparticle cross-linked aggregates. These interparticle cross-linked aggregates comprise precursor particles of substantially water-insoluble, absorbent, hydrogel-forming polymer material. This reference mainly deals with the inter-particle aggregation of these precursor particles to form aggregates. In particular, by the use of covalent cross-link bonds, the precursor particles are attached to each other to form the aggregates. There is no information in this document concerning the attachment of agglomerated particles to the substrate.

It is the object of the invention to provide ultra thin sanitary products comprising absorbents of high swellability and short-term swelling properties, which are mechanically stable under pressure even when in a swollen condition, and which swell, i.e. absorb liquid, even under pressure, which increase their liquid-storing properties even for prolonged periods of time instead of losing it, which are biodegradable and insensitive to highly osmolar solutions, in particular blood. The prefabrication of the absorbent body as a substrate matrix in combination with a swellable natural substance is novel and a decisive aspect as compared with the prior art. Preferably the substrate comprising the natural substance exist as roll goods.

The prefabricated arrangement of the substrate matrix comprising the swellable natural substance serves to achieve a construction that compensates the disadvantage of impeded swellability under pressure, and in addition, to realize a new method of manufacturing these sanitary products.

According to the invention this object is attained by an absorptive, ultra thin sanitary product comprising one layer or a plurality of layers of a natural or synthetic textile or nonwoven material and a swellable natural substance included therein and is characterized in that the swellable natural substance substantially consists of a cross-linkable, swellable polysaccharide and exists in the form of particles bonded to the surface of a solid substrate or incorporated in a substrate matrix. The particles are agglomerated having a size of 100 to 250 µm. Besides according to the invention the particles of swellable polysaccharide are attached to the substrate by way of covalent linkages.

Concerning the agglomeration of the polysaccharide materials, it is to be noted that such agglomeration is expected to improve the gel-blocking resistance, particularly when the agglomerated particles do not dis-agglomerate upon wetting. It is to be understood that on the one hand the gel-blocking phenomena are related to intrinsic gel properties, namely the gel strength. On the other hand these phenomena are also related to the particle size per se and the particle size distribution, i.e. smaller particles and wide particle size distribution lead to enhanced gel-blocking. As, by agglomeration, the particles are enlarged and the particle size distribution covers smaller ranges, the tendency to gel-blocking is decreased. Preferred polysaccharides are from the group of galactomannans, galactoglucomannans, derivates of galactomannan or galactoglucomannan, xanthenes or mixtures thereof.

Surprisingly, it has been found that the rapid swellability of the polysaccharides is maintained when the swellable-polysaccharide-based absorbent is fixed by attachment to a suitable solid substrate. The substrate preferably makes available to the swellable polysaccharide a storage volume where mechanical forces such as pressure do not impede the swellability. Simultaneously, the reduced swelling under pressure is cancelled. Presumably, this surprising effect is achieved because the substrate constitutes a kind of reinforcing matrix ensuring the swellable polysaccharide to achieve a maximum swelling volume within the substrate matrix. A favorable exploitation of the swelling space is ensured by a distribution of the swellable polysaccharide on fabric or foam within the preset swelling space.

Preferably, the quantity of swellable polysaccharide in the swelling space is 10 - 50 mg/cm³.

Such swellable polysaccharides are for instance contained in guar grain, tamarind grain or carob seed grain. Also other polysaccharide-based, natural, absorbing polymers may be used alone or in combination, such as for instance cellulose and its derivates (for instance alkyl cellulose, hydroxyalkyl cellulose, carboxymethyl cellulose), viscose fibers, gum resins (for instance gum tragacanth, arabic gum, pectin, dextran), starch and starch derivatives (such as corn, grain starch, potato starch, amylose, amylopectin, dextrin, modified starch, hydroxyethyl starch, cationic starch, starch graft polymers).

The swellable polysaccharides themselves may be used in their natural form, but also in a form chemically treated by cross-linking or self-complexing. In this connection it is to be noted that "self-complexing" is a bonding somewhat weaker than inter-particle cross-linking, but somewhat stronger than a pure hydrogen-bonding agglomeration.

It is further to be noted that the benefits of cross-linking or self-complexing show up in the so-called absorption under load (AUL) condition and not in the so-called free swell capacity (FSC). The reason is that the stability of the gel is improved at the cost of swell capacity.

Especially preferred polysaccharides are guar gum, guar gum modifications and guar gum derivatives with reactants such as diallyl urea, N,N-methylene-bis-acryloamide, N-allyl acryloamide, N,N-diallyl acryloamide, diallyl tartaric acid diamide, N,N'-ethylene-bis-methacryloamide, formaldehyde, glutaraldehyde, epichlorhydrine, ethylene oxide, carbonyldiimidazole, N-(3-dimethylaminopropyl)-N'-ethylcarbodiimide, sulphuryl chloride, phosgene. Cross-linked intramolecularly (preferably by 0.05 to 10 % by weight), these substances make swelling/absorbent bodies conferring excellent qualities to the sanitary products in which they are contained.

Preferably and depending on the desired pH, the intramolecularly cross-linked polysaccharides may be subsequently cross-linked for instance by hexahydroantimoniate ions, zirconium ions or borate ions.

Preferably the polysaccharide employed according to the invention is a cross-linked galactomannan such as for instance cross-linked gum ghatti, carob seed grain, gum tara, xanthene, and in particular cross-linked guar gum or cross-linked modifications and derivates of the latter, for instance derivatives substutited by low-molecular aliphatic, araliphatic and/or aromatic ether or ester groups, hydroxypropylated derivatives and carboxymethylated derivatives, derivatives surface-treated with a substitution rate of 0.02 to 1.4, glyoxal-treated guar gum as well as mixtures with xanthene preferably containing 0.5 to 4 % by weight of guar gum and 0.4 to 4 % by weight of xanthene, alginates, esters of alginic acid, gelatins, substituted or non-substituted celluloses, agar, carrageenan, pectin, dextran, guar gum, having at least 10 to 18 % by weight of one or a plurality of polyhydric alcohols (for instance mannite) as a stabilizer.

The absorbents and sanitary products according to the invention containing substrate-bonded polysaccharides as absorbents, serve to achieve properties surprisingly improved in various respects as compared with such absorbents and sanitary products using derivatives of polyacrylic acid or non-substrate-bonded polysaccharides.

Weakly acid as well as basic pH values may be set in the absorbents according to the invention. They are insensitive to highly osmolar liquids such as blood, urine, exudates, for instance during surgical drainage and towards bivalent ions such as calcium and magnesium ions; their performance is improved as regards their centrifuge retention capacity with urine substitute solution; they give better results in the time-controlled liquid absorption and in the time-controlled liquid retention capacity with urine substitute solution; in a swollen condition they exhibit lower susceptibility to shearing forces; they combine with various additives such as preservatives or physiological buffers; and they exhibit a higher stability under pressure, i.e. there is no or only minimal reduction of the liquid retention capacity even at an increased pressure load.

After the absorption of a certain quantity of liquid, the absorbents according to the invention exhibit the property of inducing a future maximum liquid absorption capacity, which may be denoted as material compliance. There is a steady take-up of more than 25 g of urine substitute solution per gram of cross-linked polysaccharide.

When the absorbents according to the invention are employed, physiological concentrations in particular of sodions may be used for a positive control of the liquid absorption capacity, an increased concentration of ions inducing an increased liquid absorption capacity.

Contrary to the prior art absorbents, the absorbents according to the invention have no calcium ions linking capacity, thus being insensitive to high concentrations of calcium ions as they are reached in body fluids.

Prior to being attached to or incorporated in the substrate matrix, the polysaccharide particles may exist in the form of powder, granulates or molded compounds.

The liquid absorption properties of the polysaccharide particles may be modified by urea coating.

The substrate to which the absorbent particles used according to the invention are attached can be made of a natural, half synthetic or synthetic polymer and may for instance be cellulose or a material usually used for nonwoven fabrics, in particular polypropylene, polyethylene, polyester, viscose fibers and polyurethane. Furthermore, polyhydroxy alkanoate, polycaprolactam, polylactides, polyvinyl alcohols, starch mixtures, extruded potato starch, corn starch, rice starch, hydroxypropylated starch, cellulose diacetate, regenerated cellulose have been used as suitable substrate materials and proved successful in combination with the swellable natural substance galactomannan.

The substrate may exist for instance as an open-cell or closed-cell porous structure and be constituted from - if necessary - conditioned/reinforced polyolefine fibers, conditioned/reinforced polyester fibers and/or conditioned cellulose fibers or cellulose derivate fibers, polymeric foams, textile super absorbers, foils, paper, paper containing substances or other nonwoven fabrics; for improved absorption properties, the substrates and/or the polysaccharide/substrate combination may also be equipped with a liquid supplying structural material.

Polyurethane fibers or polyurethane foams may for instance also be possible as substrate materials. When polyurethane foams are used as substrates, the polysaccharides are incorporated as a reaction component directly during the manufacture of the polyurethane foams in keeping with an embodiment of the invention; since the polysaccharides are polyhydric alcohols, they directly participate in the formation of polyurethane, which serves to achieve the desired fixing. In keeping with another embodiment, the polysaccharide powder is poured in after the polymerization of the polyurethane foam. Both approaches can be combined.

A suitable substrate structure is formed by at least one nonwoven fabric and at least one film, swelling volume being produced therebetween by gasfilled bubbles. The bubbles form a uniform pattern that depends on the size of the bubbles and ensures the unpressurized swelling of the natural substances.

A further substrate structure is formed by honeycombs in which the swellable natural substance is fixed or included. The walls of the honeycombs may be gasfilled structures. A honeycomb structure with gasfilled walls may be produced from films by deep drawing. Upwardly, this absorbent body is defined by a nonwoven cover.

The use of biodegradable film and biodegradable nonwoven in combination with the swellable polysaccharides gives a compostable absorbent body.

The upper layer placed on top of the absorbent body may for instance be a fibrous web, of which the fibers extend downwardly in the direction towards the layer of particles of swellable, cross-linked or/and derivatized polysaccharide attached to a substrate. The lower storage layer can for instance be a fibrous fabric that contains the particles according to the invention of swellable cross-linked or/and derivatized polysaccharide attached to a substrate or serves itself as a substrate for the particles of swellable, cross-linked or/and derivatized polysaccharide.

Suitably, the particles of swellable, cross-linked or/and derivatized polysaccharide are uniformly distributed in the substrate structure.

In keeping with a further embodiment, the sanitary product according to the invention may have a layer of substrate structures which comprises areas of high concentration of particles of swellable, cross-linked or/and derivatized polysaccharide, the substrate structure between the areas of high concentration having no or only minor concentrations of particles of swellable, cross-linked or/and derivatized polysaccharide.

When the substrate exists in the form of a three dimensional fibrous or nonwoven fabric, it is to exercise not only carrying and supporting functions for the swellable polysaccharide, but to exhibit also vertical and horizontal liquid conducting and absorbing functions, in particular when the layer of three-dimensional substrate material serves as the only layer or as one of the layers constituing the sanitary product, for instance as a layer of a diaper structure.

When the polysaccharides are attached to a substrate which itself exists in the form of particles, or when the combination according to the invention of swellable polysaccharide and substrate is to exist in a comminuted form, then the substrate substances are employed preferably in the form of short fibers. However, it is also possible to use a three-dimensional substrate material or a substrate in the form of longer fibers, fixing the polysaccharides thereon, and subsequently to comminute the product thus obtained or to transform it into short fibers.

In order to achieve optimal results in light of the surprisingly good qualities mentioned before of the absorbents and sanitary products according to the invention, the selection of the substrate substances, and in particular also the combination of the polysaccharide and substrate employed according to the invention, is of major importance.

The development of an absorbent body in which the swellable natural substance is attached to a liquid supplying substrate permits to do without the manufacturing methods so far used for the production of incontinence products and sanitary napkins. Contrary to methods in which the polysaccharide is incorporated as granulate material in the cellulose body to make the absorbent body, a considerable manufacturing advantage can be achieved by rolls or sheets of the absorbent body being prepared. Advantages reside in the abandonment of methods of super absorbers being poured in and of pulping, which is accompanied by an acceleration in the manufacture of the products, a facilitated variability of the types of product and in new structural features.

Otherwise, the design of the sanitary products according to the invention, such as for instance of diapers, and the method of manufacturing them may correspond to structures and methods known for products of the generic type. The only important thing is that the sanitary products comprise at least one layer consisting of, or containing, an absorbent structure according to the invention, i.e. a swellable polysaccharide attached to a solid substrate. The absorbent according to the invention may again exist in a form attached to a suitable substrate.

In keeping with a further embodiment, the film material may form a bag or an open cavern, inside of which an absorbent according to the invention, i.e. swellable polysaccharide particles attached to a substrate, and, as the case may be, additional particles not attached to a substrate are contained.

Preferably, the swellable polysaccharide is incorporated already in the course of the process of manufacturing the substrate material. New ways of manufacture of the ultimate product, such as diapers, result from this manufacturing process.

In the sanitary product/absorbent structure according to the invention, the swellable polysaccharide particles are attached to the substrates by way of a covalent linkage generated by chemical cross-linking.

For covalent linkages to form through chemical cross-linking, a divalent cross-linking agent is preferably used, such as a cross-linking agent known for the combination of enzymes with a suitable substrate, for instance glyoxal. The cross-linking can take place in a manner known per se.

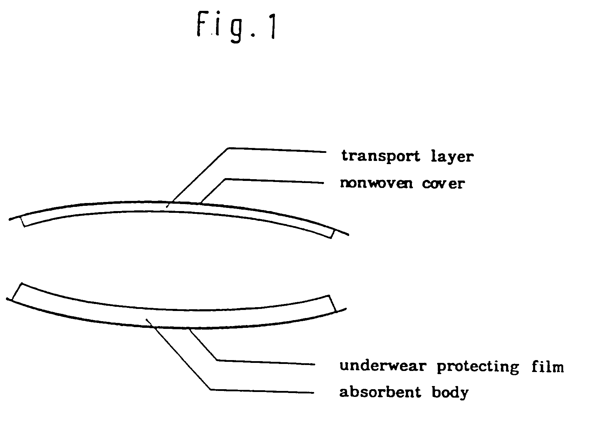

Suitably, the sanitary product according to the invention has an external structure which, in cross-sectional area, consists of four function elements as illustrated in Fig. 1 of the attached drawing.

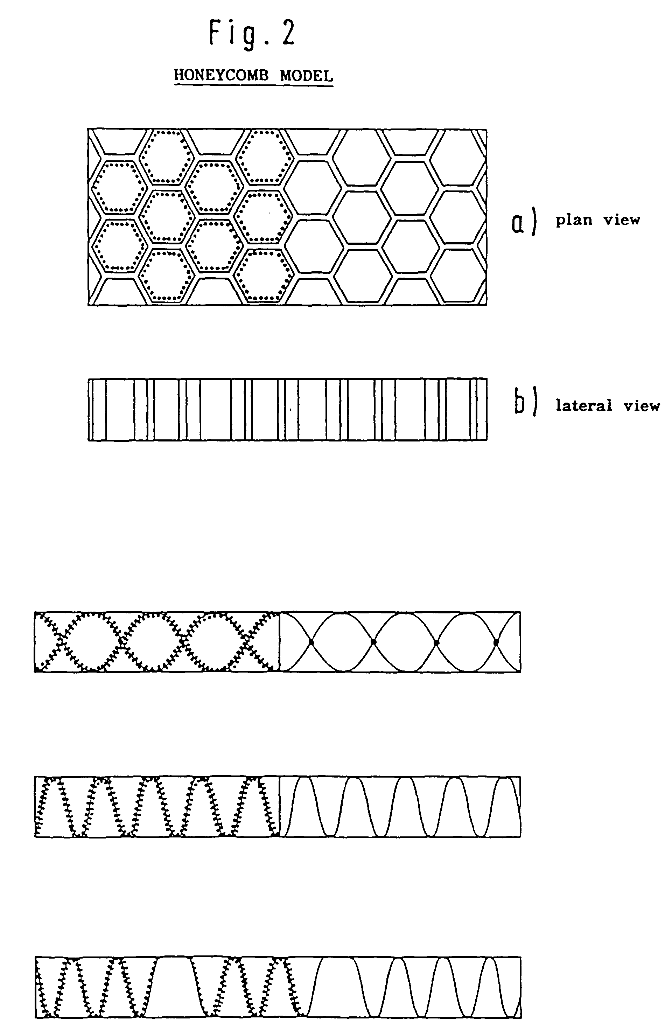

- Fig. 2: illustrates the honeycomb design of the substrate.

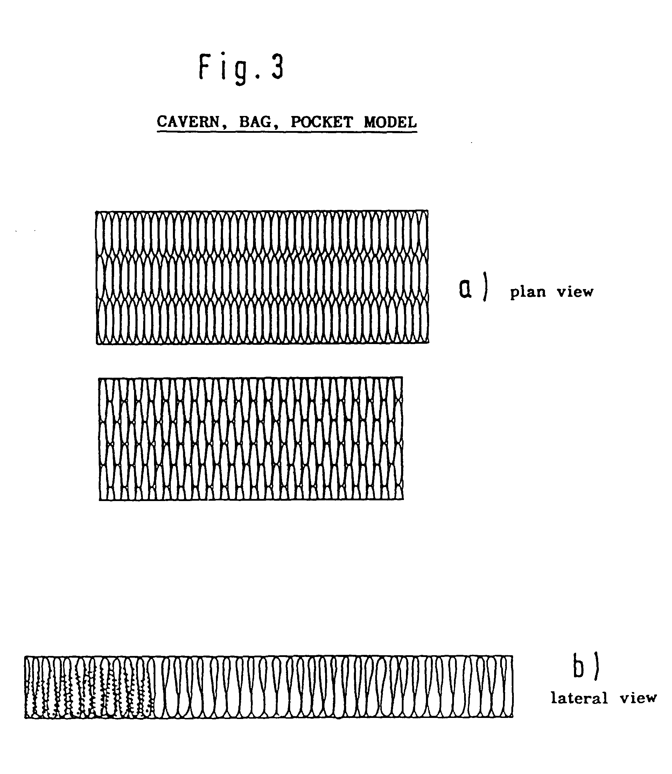

- Fig. 3: illustrates the bag or cavern design of the substrate.

The first element is a naturally breathing and water vapour permeable underwear-protecting film contacting the clothes of the user and made of a reliable PP/PE material (no-rustle and of low noise) or of a biodegradable film material of derivates of copolymers of cellulose or starch, polyhydroxy alkanoate suitably in the range of 10 to 40 µm preferably 15 to 35 µm.

As a result of the combination of the absorbent body and the underwear-protecting film, this film is an integral component of the roll-goods-type prefabricated absorbent body. This is an alternative of the diaper and napkin structures produced from individual elements.

The second element is the absorbent body comprising the substrate material and the polysaccharide, i.e. the absorbent structure according to the invention.

Preferably, the substrate matrix for the swellable polysaccharides passes the absorbed liquid thoughout to swelling bodies and constitutes the storage layer.

As a third element, a liquid distributing zone (transport layer) is situated on top of the storage layer. This function zone covers the entire horizontal storage surface, making liquid available to the storage zone. Ideally, this zone may consist of oriented, cross-linked cellulose fibers or of dry airlaid cellulose substances comprising hydrophilicized nonwoven components.

In accordance with the invention, the substances of the distribution zone are combined with an acquisition zone (nonwoven cover) which constitutes the fourth element.

As for the acquisition zone or absorbing diaper zone, extremely hydrophilicized, dry airlaid and PP nonwoven substances have proved successful. This construction gives a modular diaper structure of which the layers can be prefabricated as roll material.

Apart from the liquid absorbent area, all the external substances have liquid repellent (hydrophobic) properties.

The sanitary products have an absorbent body differing in size depending on their application.

Therefore, the sanitary products according to the invention and the absorbents according to the invention are extraordinarily suitable for sanitary and medical products, such as baby diapers, sanitary napkins, surgical pads, bandages, bed pads, dressing material, veterinary products, diagnostic kit carriers and incontinence products.

Further possible application also result from a combination with additives and/or agents as may exist chemically attached to the absorbent according to the invention or physically mixed with it, and which, given different chemical or physical conditions, exhibit for instance a pharmacological or cosmetic effect corresponding to the agent or additive.

### EXAMPLE 1

In accordance with the invention an absorbent body was produced having the overall size of 70 x 20 cm, of which the overall thickness ranges from 3 to 7 mm and which in an unloaded, swollen condition, does not exceed 10 mm of overall body thickness. The absorbent consisted of guar gum cross-linked with glyoxal and having the form of particles of an average diameter of 170 µm fixed on dry airlaid nonwoven by means of a hot-melt adhesive. The absorption volume of the diaper ranges between 400 and 900 ml for different kinds of applications, given a constituent amount of swelling bodies in the substrate material of between 10 and 50 mg/cm³ of volume of a dry diaper. Rewetting ratios of less than 0.1 g/g of swellable polysaccharide at storage rates of 30 - 40 seconds for 150 ml of urine substitute solution (HEL) were found. Multiple actuation (3 x 100 ml) showed stable rewetting ratios of less than 0.1 g/g of swellable natural substance at absorption rates of 90 to 180 seconds.

The substrate and/or the polysaccharides or the swellable polysaccharide attached to a substrate or the finished sanitary product may be completed by additives such as preservatives, for instance potassium sorbate, p-hydroxybenzoic acids, imidazolidinyl urea, salicylic acid, trichlorcarbanilide, polyhexamethylene guanidine/guanite, which stabilize the substrate and the polysaccharides against microbiological decomposition and which additionally provide for dermatologic protection of the skin without reducing the swelling and absorbing performance. Further additives may be physiological buffers such as citrates, lactates, malates or phosphates, which stablize the substrate and the polysaccharides in a medium kind to the skin. Further to their swelling properties, some of the polysaccharides used according to the invention have buffering properties in an acid medium.

### EXAMPLE 2

### Introduction

This example shows the positive effect of agglomerating and chemically treating (e.g. cross-linking or preparing a self-complexing swellable material) polysaccharides concerning their absorbency characteristics. Guar grain is the basic material used here.

Native guar grain is almost totally soluble in water. Owing to the soluble constituents it is only of limited use in sanitary products.

However, the high FSC (free swell capacity) in the range of 45 g HEL/g for industrially produced, agglomerated guar (Meyhall ID 310) argues in favor of the use of guar. Laboratory tests have shown that the soluble constituents can be strongly decreased or entirely eliminated by physical cross-linkage. The cross-linkage is accompanied by the occurrence of an AUL (absorption under load) increase.

The physical cross-linking of native guar is only possible because of the latter's special structure. Guar consists of β-1,4 glycosidically combined mannose units carrying, on a statistical average, an α -1,6 combined galactose unit on every second group. Owing to the vicinal hydroxyl groups (cis position) of the mannose, guar is especially suitable for the formation of hydrogen bridges. This is also a reason for the capability of physical cross-linking (chelation) with borates, antimonates, zirconates and similar compounds.

The requirements for the physical cross-linking of guar are technically simple (low costs). The following steps are necessary:

### 1st Step

### Pretreatment

Guar is to be agglomerated in a mechanically produced fluidized bed (mixer) by the addition of NaOH solution such that its swelling properties correspond to the agglomerated guar ID 310 (pneumatically produced fluidized bed) of Meyhall. The important advantage of the agglomeration in a mixer as compared to a fluidized bed resides in that the educt is not only agglomerated but simultaneously compacted. This results in a product of higher grain stability and more favorable mechanical properties.

An agglomeration of guar is only possible in the alkaline condition, since no powdery agglomerates can be formed in the neutral medium as a result of the strong gluing of guar.

### 2nd Step

### Preparation of self-complexing guar

Agglomerated guar is suitable for use as a swelling body only to a limited extent, since no cross-linking occurs during the agglomeration, the guar thus not being able to swell. The agglomerated guar must still be physically cross-linked for instance by the addition of borate to obtain a gel-forming product.

It is not necessary additionally to add an alkaline source (for instance NaOH), agglomerated guar and borate already being basic. It is sufficient to mix the borate homogeneously into the agglomerated guar. The product thus obtained is not a cross-linked guar. It is a self-complexing guar cross-linking to form a gel only when liquid is added.

### 3rd Step

### Formulation

By the addition of suitable aids the performance characteristics (swelling properties) of guar can still be improved. Laboratory tests have shown that hydrophilic aerosil COK 84 (84 % aerosil 200 + 16 % pyrogenic aluminum oxide) is especially suitable. On the one hand it provides for the swelling body to be wetted promptly and on the other hand it results in an improvement of the swelling properties. Because of the low bulk density (large volume), aerosil also works as a spacer (anti-blocking agent) and pressure protection matrix, since aerosil can hardly be compacted.

### 4th Step

### Cross-linked guar

Cross-linking is a further possibility to produce a swelling body from native guar. Contrary to the preparation according to Step 2, the guar is agglomerated and cross-linked in a single step. An NaOH/borate solution is jetted into the mixer. The product is then in fact a physically cross-linked, insoluble guar. The laboratory tests show that "mild" drying (low temperatures; long drying periods) result in good performance characteristics.

### Implementation

3 L VE water is prepared in a suitable vessel (stirred tank), after which 37.5 g sodium hydroxide is added. The solution is stirred until the NaOH pellets have completely dissolved. The sodium hydroxide solution is then transferred into the receiver.

Now, 15 kg native guar grain (CSA 200/50) is added into the mixer, the mixer is switched on, and the sodium hydroxide solution is jetted at overpressure from the receiver into the mixing chamber, whereby the agglomeration takes place.

After termination of the agglomeration, two samples are taken which are dried in a shelf drier (3 h 80° C/designation of sample V1/P1H) and a laboratory fluidized bed (30 min 80° C/designation of sample V1/P1W).

750 g aerosil COK 84 are added and mixed homogeneously. Again two samples (V1/ and V1/P2W) are taken and dried as above.

Then the product is dried by heated air of 80° C being blown into the mixer, and a sample (V1/P3) is taken after termination of the drying. The dried product is then added by 150 g sodium tetraborate decahydrate (1 % referred to the quantity of guar grain employed), mixing takes place, and the final product (V1) is drawn off.

**Table 1**

| design. of sample | TG [Gew.%] | FSC [gHEL/g] | CRC [gHEL/g] | AUL20 [gHEL/g] | AUL50 [gHEL/g] | AUL70 [gHEL/g] |
|---|---|---|---|---|---|---|
| CSA 200/50 | 90,4 | 36,1 | 30,5 | 5,8* | 4,8* | n. b. |
| ID 310 | 90,0 | 40,3 | 28,4 | 6,9* | 5,4* | n. b. |
| V1/P1W | 98,4 | 37,0 | 30,8 | 8,6* | 8.5* | 8,7* |
| V1/P1H | 96,5 | 33,1 | 30,1 | 7,4* | 8,4* | 8,5* |
| V1/P2W | 94,4 | 40,1 | 32,7 | 10,3 | 9,2 | 10,0 |
| V1/P2H | 91,4 | 36,5 | ∼30 | 10,2 | 10,3 | 10,2 |
| V1/P3 | 90,7 | 32,0 | 26,9 | 11,4 | 10,4 | 10,6 |
| V1 | 90,7 | 31,4 | 27,9 | 13,0 | 12,4 | 10,0 |
| Table 1: performance characteristics Test 1 | | | | | | |

| | | | | | | |
|---|---|---|---|---|---|---|
| * gel blocking HEL: urine substitute solution n.b.: not determined | | | | | | |

Table 1 shows that the AUL increases throughout the course of reaction. The various methods of sample drying do not affect the performance characteristics.

The FSC is increased by the agglomeration and addition of aerosil, but there is a strong decrease as a result of the drying in the mixer (fluidized-bed drying in a mechanically produced fluidized bed). During sample drying, the FSC exhibits some dependence on the drying method, the values of shelf drying being below those of fluidized-bed drying (pneumatic) by approximately 4 g/g in parallel.

Moderate gel blocking occurs in the educt, as in the samples V1/P1. All samples taken after the addition of aerosil are free from gel blocking. Consequently, aerosil may be used as an anti-blocking agent.

In the samples to which no borate was added (V1/P1 to V1/P3) it is remarkable that there is no substantial change of the AUL with the increase of pressure. But in the product, the AUL decreases with the increase of pressure similarly to polyacrylates. This is due to the fact that the swelling balance had not yet been reached in the samples V1/P1 to V1/P3, the sample thus not being able to "deswell" (no decrease of the AUL possible): As a result of the cross-linking of the guar, the "swelling curve" shifts such that the swelling balance is attained and "deswelling" is possible. As seen in Table 1, this is true for the product. Consequently, it is to be assumed that the procuct V1 is a cross-linked and self-complexing guar.

A further indication of cross-linking is the decrease of the FSC from the educt to the product.

### EXAMPLE 3

### Cross-linking of carboxymethyl starch (CMS)

Carboxymethyl starch ES type II (ESII) of the German company Emsland-Starke was used as base material for the cross-linking reactions carried out.

The cationically attacking compound polyethylene imine (PEI) was used for the cross-linking of the polyanion ESII in suspension.

For the purpose of cross-linking, 10.5 g CMS were weighed into a roundbottomed flask of 250 ml and added by a solution of 0.1 or 0.5 g of a cross-linking agent / 5 ml water / 45 ml isopropanol. After the uniform wetting of the sample (0.5 to 1 h at ambient temperature), vacuum evaporating was performed in a rotary evaporator at 80° C of bath temperature until drying. This was accompanied by repeated and strong agitation to keep the preparation pourable without clogging.

The products were characterized without any aftertreatment and cleaning steps. Table 2 is a collection of the type and quantity used of the cross-linking agent and of the swelling behavior of the samples.

**Table 2:**

| Cross-linking of CMS (ES type II) in isopropanol/water and characterisation of the products with regard to the absorption properties towards urine substitute solution (HEL) (addition of the cross-linking agent in the mix by 45 ml i-PrOH/5ml H₂O per 10 g CMS at 20°C, vacuum drying at 80°C) | | | | | | |
|---|---|---|---|---|---|---|
| No. of sample | Cross-linking agent (VM) | gVM/10g CMS | Sorption capacity (gHEL/gCMS) | | | |
| | | | FSC | CRC | AUL20 | AUL50 |
| CMS | no | no | 21 | 14.8 | 5.5 | 5.0 |
| U4 | PEI (50%) | 0.1 | 24 | 13 | 6.9 | 5.5 |
| U5 | PEI (50%) | 0.5 | 22.6 | 13.3 | 6.1 | 5.4 |

As is shown by table 2, the AUL values of CMS cross-linked by PEI are significantly increased as compared to the corresponding values of the basic CMS. This goes along with a slight decrease of the CRC value.

## Claims

1. Absorptive, ultra thin sanitary products comprising one layer or a plurality of layers of a textile material and a swellable natural substance included therein, wherein the swellable natural substance substantially consists of a cross-linkable, swellable polysaccharide and exists in the form of particles attached to the surface of a solid prefabricated substrate or incorporated in a prefebricated substrate matrix, **characterized in that** the particles are agglomerated, having a size of 100 to 250 µm and **in that** the particles of swellable polysaccharide are attached to the substrate by way of covalent linkages.

2. A sanitary product according to claim 1, **characterized in that** the polysaccharide particles are selected from the group of galactomannans, galactoglucomannans, derivates of galactomannans or galactoglucomannans, xanthenes or mixtures thereof.

3. A sanitary product according to claim 1 or 2, **characterized in that** the particles of swellable polysaccharide exist in the form of a powder, granulates or flakes.

4. A sanitary product according to one of claims 1 to 3, **characterized in that** the substrate is formed of a natural, half synthetic or synthetic polymer.

5. A sanitary product according to claim 4, **characterized in that** the substrate consists of cellulose.

6. A sanitary product according to one of the claim 1 to 5, **characterized in that** the particles of swellable polysaccharide are attached to the substrate by way of a divalent cross-linking agent

7. A sanitary product according to claim 6, **characterized in that** the divalent cross-linking agent is glyoxal.

8. A sanitary product according to one of claims 1 to 7, **characterized in that** it comprises at least two layers, of which an upper layer is such that liquid can rapidly spread in the direction towards a layer of a swellable natural substance, but also in directions parallel to the surface of the layers, and of which the lower layer consists of particles of swellable polysaccharide attached to the substrate.

9. A sanitary product according to claim 8, **characterized in that** the upper layer is a fibrous web, of which the fibers extend downward in the direction towards the layer of particles of swellable polysaccharide attached to the substrate.

10. A sanitary product according to claim 8 or 9, **characterized in that** the lower layer is a fibrous fabric.

11. A sanitary product according to claim 8 or 9, **characterized in that** it consists of a layer of a substrate structure, in which the particles of swellable polysaccharide are uniformly distributed.

12. A sanitary product according to one of claims 1 to 7, **characterized in that** it consists of a layer of a substrate structure, which comprises areas of high concentration of particles of swellable polysaccharide, the substrate structure between the areas of high concentration of particles of swellable polysaccharide having no or only a low concentration of particles of swellable polysaccharide.

13. A sanitary product according to claim 12, **characterized in that** one layer gives, or a plurality of layers combine to give, a thickness of 1 to 10 mm.

14. A sanitary product according to one of claims 1 to 13, **characterized in that** the polysaccharide particles exist as such in their natural form and/or a form chemically treated by cross-linking, self-complexing or the like.

15. A sanitary product according to claim 14, **characterized in that** the particles of swellable polysaccharides consist of or contain guar gum.

16. A sanitary product according to claim 14 or 15, **characterized in that** the polysaccharides are cross-linked by means of antimoniates or zirconium salts.

17. A sanitary product according to one of the preceding claims, **characterized in that** it exists as a windable web.

18. A sanitary product according to one of the preceding claims, **characterized in that** it further contains additives which provide for dermatologic protection of the skin.

19. A sanitary product according to one of the preceding claims, **characterized in that** the polysaccharide particles are superficially urea-modified.

## Patentansprüche

1. Saugfähige ultradünne Hygieneprodukte, welche eine oder mehrere Schichten aus einem Textilmaterial und eine darin eingeschlossene quellfähige natürliche Substanz enthalten, wobei die quellfähige natürliche Substanz im wesentlichen aus einem vernetzbaren quellfähigen Polysaccharid besteht und in der in Form von Teilchen, welche an der Oberfläche eines festen, vorgefertigten Substrats fixiert oder innerhalb einer vorgefertigten Substratmatrix eingebunden sind, vorliegt, **dadurch gekennzeichnet, dass** die Teilchen agglomeriert sind und eine Größe von 100 bis 250 µm aufweisen und dass die Teilchen aus quellfähigem Polysaccharid an das Substrat über kovalente Bindungen gebunden ist.

2. Hygieneprodukt nach Anspruch 1, **dadurch gekennzeichnet, dass** die Polysaccharid-Teilchen aus der Gruppe der Galactomannane, Galactoglucomannane, Derivate der Galactomannane oder der Galactoglucomannane, Xanthane oder Mischungen davon ausgewählt sind.

3. Hygieneprodukt nach Anspruch 1 oder 2, **dadurch gekennzeichnet, dass** die Teilchen aus quellfähigem Polysaccharid in der Form eines Pulvers, Granulats oder von Flocken vorliegen.

4. Hygieneprodukt nach einem der Ansprüche 1 bis 3, **dadurch gekennzeichnet, dass** das Substrat aus einem natürlichen, halbsynthetischen oder synthetischen Polymer gebildet ist.

5. Hygieneprodukt nach Anspruch 4, **dadurch gekennzeichnet, dass** das Substrat aus Zellulose besteht.

6. Hygieneprodukt nach einem der Ansprüche 1 bis 5, **dadurch gekennzeichnet, dass** die Teilchen aus quellfähigem Polysaccharid an das Substrat über ein divalentes Vernetzungsmittel gebunden ist.

7. Hygieneprodukt nach Anspruch 6, **dadurch gekennzeichnet, dass** das divalente Vernetzungsmittel Glyoxal ist.

8. Hygieneprodukt nach einem der Ansprüche 1 bis 7, **dadurch gekennzeichnet, dass** es mindestens zwei Schichten umfasst, von denen eine obere Schicht so ist, dass sich Flüssigkeit rasch in der Richtung gegen eine Schicht aus einer quellfähigen natürlichen Substanz, aber auch in zu der Oberfläche der Schichten parallelen Richtungen ausbreiten kann, und von denen die untere Schicht aus Teilchen aus quellfähigem Polysaccharid besteht, welche am Substrat fixiert sind.

9. Hygieneprodukt nach Anspruch 8, **dadurch gekennzeichnet, dass** die obere Schicht eine faserige Bahn ist, deren Fasern sich nach unten in Richtung auf die Schicht aus Teilchen aus quellfähigem Polysaccharid, welche am Substrat fixiert sind, erstrecken.

10. Hygieneprodukt nach Anspruch 8 oder 9, **dadurch gekennzeichnet, dass** die untere Schicht ein faseriges Textilmaterial ist.

11. Hygieneprodukt nach Anspruch 8 oder 9, **dadurch gekennzeichnet, dass** es aus einer Schicht einer Substratstruktur besteht, in der die Teilchen aus quellfähigem Polysaccharid gleichmäßig verteilt sind.

12. Hygieneprodukt nach einem der Ansprüche 1 bis 7, **dadurch gekennzeichnet, dass** es aus einer Schicht einer Substratstruktur besteht, die Zonen hoher Konzentration von Teilchen aus quellfähigem Polysaccharid enthält, wobei die Substratstruktur zwischen den Zonen hoher Konzentration an Teilchen aus quellfähigem Polysaccharid keine oder nur eine geringe Konzentration an Teilchen aus quellfähigem Polysaccharid aufweist.

13. Hygieneprodukt nach Anspruch 12, **dadurch gekennzeichnet, dass** eine Schichte, oder mehrere Schichten zusammen, eine Dicke von 1 bis 10 mm ergbit.

14. Hygieneprodukt nach einem der Ansprüche 1 bis 13, **dadurch gekennzeichnet, dass** die Polysaccharid-Teilchen als solche in ihrer natürlichen Form und/oder in einer Form vorliegen, in welcher sie durch Vernetzung, Eigenkomplexierung oder dergleichen chemisch behandelt vorliegen.

15. Hygieneprodukt nach Anspruch 14, **dadurch gekennzeichnet, dass** die Teilchen aus quellfähigem Polysaccharid aus Guar-Gummi bestehen oder diesen enthalten.

16. Hygieneprodukt nach Anspruch 14 oder 15, **dadurch gekennzeichnet, dass** die Polysaccharide mittels Antimonaten oder Zirkoniumsalzen vernetzt sind.

17. Hygieneprodukt nach einem der vorhergehenden Ansprüche, **dadurch gekennzeichnet, dass** es als eine aufwickelbare Bahn vorliegt.

18. Hygieneprodukt nach einem der vorhergehenden Ansprüche, **dadurch gekennzeichnet, dass** es zusätzlich Additive enthält, die einen dermatologischen Hautschutz verleihen.

19. Hygieneprodukt nach einem der vorhergehenden Ansprüche, **dadurch gekennzeichnet, dass** die Polysaccharid-Teilchen an der Oberfläche mit Harnstoff modifiziert sind.

## Revendications

1. Produit hygiénique absorbant, ultramince comprenant une ou plusieurs couches d'un matériau textile et comportant une substance naturelle gonflable, dans lequel la substance naturelle gonflable est essentiellement constituée d'un polysaccharide apte à être réticulé, gonflable, et existe sous la forme de particules fixées à la surface d'un substrat préfabriqué solide, ou incorporées dans une matrice préfabriquée de substrat, **caractérisé en ce que** les particules sont agglomérées, ayant une dimension de 100 à 250 µm, et **en ce que** les particules de polysaccharide gonflable sont fixées au substrat grâce à des liaisons covalentes.

2. Produit hygiénique selon la revendication 1, **caractérisé en ce que** les particules de polysaccharide sont choisies parmi le groupe des galactomannanes, des galactoglucomannanes, des dérivés de galactomannanes ou de galactoglucomannanes, des xanthènes ou leurs mélanges.

3. Produit hygiénique selon la revendication 1 ou 2, **caractérisé en ce que** les particules de polysaccharide gonflable existent sous la forme d'une poudre, de granulés ou de flocons.

4. Produit hygiénique selon l'une des revendications 1 à 3, **caractérisé en ce que** le substrat est formé d'un polymère naturel, semi synthétique ou synthétique.

5. Produit hygiénique selon la revendication 4, **caractérisé en ce que** le substrat est constitué de cellulose.

6. Produit hygiénique selon l'une des revendications 1 à 5, **caractérisé en ce que** les particules de polysaccharide gonflable sont fixées au substrat à l'aide d'un agent de réticulation divalent.

7. Produit hygiénique selon la revendication 6, **caractérisé en ce que** l'agent de réticulation divalent est le glyoxal.

8. Produit hygiénique selon l'une des revendications 1 à 7, **caractérisé en ce qu'**il comprend au moins deux couches dont une couche supérieure est telle que les liquides peuvent s'étendre rapidement dans la direction allant vers une couche de la substance naturelle gonflable, mais aussi dans des directions parallèles à la surface des couches, et dont la couche inférieure est constituée de particules de polysaccharide gonflable fixées au substrat.

9. Produit hygiénique selon la revendication 8, **caractérisé en ce que** la couche supérieure est une couche fibreuse dont les fibres s'étendent vers le bas dans la direction allant vers la couche de particules de polysaccharide gonflable fixées au substrat.

10. Produit hygiénique selon la revendication 8 ou 9, **caractérisé en ce que** la couche inférieure est un tissu fibreux.

11. Produit hygiénique selon la revendication 8 ou 9, **caractérisé en ce qu'**il est constitué d'une couche d'une structure de substrat, dans lequel les particules de saccharide gonflable sont réparties uniformément.

12. Produit hygiénique selon l'une des revendications 1 à 7, caractérisé en qu'il est constitué d'une couche d'une structure de substrat, qui comprend des zones de concentration élevée de particules de polysaccharide gonflable, la structure de substrat située entre les zones de concentration élevée de particules de polysaccharide gonflable n'ayant pas de concentration de particules de polysaccharide gonflable, ou n'ayant qu'une faible concentration de particules de polysaccharide gonflable.

13. Produit hygiénique selon la revendication 12, **caractérisé en ce qu'**une couche donne ou **en ce que** plusieurs couches se combinent pour donner une épaisseur de 1 à 10 mm.

14. Produit hygiénique selon l'une des revendications 1 à 13, **caractérisé en ce que** les particules de polysaccharide existent en tant que telles sous leur forme naturelle et/ou sous une forme chimiquement traitée par réticulation, auto complexion, ou analogue.

15. Produit hygiénique selon la revendication 14, **caractérisé en ce que** les particules de polysaccharide gonflable sont constituées de gomme guar ou contiennent de la gomme guar.

16. Produit hygiénique selon la revendication 14 ou 15, **caractérisé en ce que** les polysaccharides sont réticulés à l'aide d'antimoniates ou de sels de zirconium.

17. Produit hygiénique selon l'une des revendications précédentes, **caractérisé en ce qu'**il existe en tant que nappe pouvant être enroulée.

18. Produit hygiénique selon l'une des revendications précédentes, **caractérisé en ce qu'**il contient, en outre, des additifs qui fournissent une protection dermatologique de la peau.

19. Produit hygiénique selon l'une des revendications précédentes, **caractérisé en ce que** les particules de polysaccharide sont superficiellement modifiées par l'urée.
